(19) Europäisches Patentamt
European Patent Office
Office européen des brevets

(11) Veröffentlichungsnummer: **0 030 278**
**B1**

(12) # EUROPÄISCHE PATENTSCHRIFT

(45) Veröffentlichungstag der Patentschrift: **06.06.84**

(51) Int. Cl.³: **C 09 B 63/00,** C 09 B 67/24 // A23L 1/275, A61K 7/021

(21) Anmeldenummer: **80106911.3**

(22) Anmeldetag: **08.11.80**

(54) **Verfahren zur Herstellung von wasserunlöslichen organischen Farbstoffen.**

(30) Priorität: **08.12.79 DE 2949440**

(43) Veröffentlichungstag der Anmeldung: **17.06.81 Patentblatt 81/24**

(45) Bekanntmachung des Hinweises auf die Patenterteilung: **06.06.84 Patentblatt 84/23**

(84) Benannte Vertragsstaaten: **BE CH FR GB IT LI NL**

(56) Entgegenhaltungen:
**DE - A - 2 338 759**
**DE - A - 2 527 571**
**FR - A - 342 903**
**FR - A - 930 768**
**GB - A - 1 456 582**

(73) Patentinhaber: **Degussa Aktiengesellschaft, Weissfrauenstrasse 9, D-6000 Frankfurt am Main 1 (DE)**

(72) Erfinder: **de Ahna, Heinz Dieter, Dr., Dipl.-Chem., Wolfg.-Borchert-Strasse 11, D-6073 Egelsbach (DE)**
Erfinder: **Kleinschmit, Peter, Dr.,Dipl.-Chem., Wildaustrasse 19, D-6450 Hanau 9 (DE)**

**EP 0 030 278 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von wasserunlöslichen organischen Farbstoffen, insbesondere für die Anwendung auf dem Kosmetik-, Lebensmittel und Arzneimittelsektor, mit in kristallines Aluminiumoxid eingelagerten wasserlöslichen organischen Farbstoffen.

An Farbstoffe auf dem Gebiet der Kosmetik, Lebensmittel oder Arzneimittel, die chemisch gesehen meist entweder zur Reihe der Azofarbstoffe (synthetische Farbmittel) oder zur Gruppe der Carotinoide (natürliche Farbmittel) gehören, werden besondere Anwendungskriterien bezüglich ihrer physiologischen Unschädlichkeit gestellt. Die zunehmende Verschärfung dieser Anwendungskriterien durch behördliche Maßnahmen hat dazu geführt, daß die Palette der zur Verfügung stehenden Farbmittel in den letzten Jahren immer kleiner wurde. Auch die wenigen noch verbliebenen Farbmittel geraten nach neueren Befunden über das Auftreten von Inkompatibilitäten immer mehr in das Kreuzfeuer der Kritik.

Man versuchte in der Vergangenheit dieses Problem in der Weise zu lösen, daß man wasserlösliche Farbstoffe durch sogenannte Farblacke ersetzte, die eine geringere Löslichkeit in Wasser und physiologischen Säften aufweisen. Diese Farblacke entstehen nach folgender Gleichung:

$$\text{Na-Farbstoff} + Al_2(SO_4)_3 \rightarrow \text{Al-Farbstoff} + Na_2SO_4.$$

Es hat sich jedoch herausgestellt, daß auch diese Farblacke aufgrund der doch noch beträchtlichen Löslichkeit insbesondere in physiologischen Säften (z. B. Magensaft) ebenfalls nicht mehr den verschärften Kriterien genügen.

Aus der DE-OS 2 338 759 ist ein Verfahren bekannt, organische Farbstoffe in die Gitter von farblosen anorganischen Trägermaterialien, wie Aluminiumoxid, einzulagern, indem das Trägermaterial in einer Färbeflotte des Farbstoffes bei einem bestimmten pH-Wert suspendiert und bei 10—140°C angefärbt wird. Diese Farbpigmente sind gegen physiologische Säfte aber ebenfalls nicht ausreichend beständig.

Es war daher Aufgabe der vorliegenden Erfindung, ein Verfahren zur Herstellung von wasserunlöslichen organischen Farbstoffen zu finden, insbesondere zur Verwendung als Kosmetik-, Lebensmittel- und Arzneimittel-Farbstoff, mit in kristallines Aluminiumoxid eingelagerten wasserlöslichen organischen Farbstoffen, die gegen physiologische Säfte beständig sind.

Diese Aufgabe wurde erfindungsgemäß dadurch gelöst, daß aus einer Lösung eines Aluminiumsalzes mit dem Farbstoff Aluminiumhydroxid ausgefällt und der abgetrennte Niederschlag bei Temperaturen zwischen 250 und 500°C geglüht wird.

Es wurde gefunden, daß z. B. Azofarbstoffe, die auf diese Weise in kristallinem Aluminiumoxidhydrat eingebettet bzw. suspendiert werden, die verschärften Anwendungsbedingungen erfüllen.

Besonders bewährt hat sich ein Verfahren, bei dem aus einer homogenen Lösung von Farbstoff und eines Aluminiumsalzes die Fällung von Aluminiumhydroxid erfolgt. Das bei diesem Fällprozeß entstehende Produkt wird getrocknet und bei Temperaturen zwischen 250 und 500°C geglüht. Überraschend ist, daß der im Kristall eingebettete bzw. verteilte Farbstoff diese Temperaturbehandlung übersteht, während der reine Farbstoff verändert bzw. zerstört würde.

Nach dem Glühprozeß wird das Produkt gewaschen. Der Waschprozeß kann mit Wasser oder auch verdünnten Mineralsäuren (z. B. Salzsäure, Phosphorsäure o. a.) erfolgen. Die auf diese Weise erhaltenen Farbstoffe verhalten sich gegenüber Wasser oder Körpersäften wie reines kristallines Aluminiumhydroxid, d. h. sind weitestgehend unlöslich.

Folgende Beispiele sollen die erfindungsgemäßen Verfahren näher erläutern:

Beispiel 1

200 ml Na-Aluminatlösung werden mit 1 g Amaranth-Farbstoff versetzt und unter Rühren 100 ml Salzsäure (30—33%ig) zugegeben. Der voluminöse Niederschlag wird abfiltiert und bei 110°C getrocknet. Der Trockenkuchen wird feingemahlen und bei 370°C 10 min geglüht. Durch Waschen mit Wasser wird überschüssiger Farbstoff entfernt. Das nach der Trocknung erhaltene blauviolette Farbmittel ist unlöslich in Wasser oder künstlichem Magensaft.

Beispiel 2

100 g $Al_2(SO_4)_3 \cdot 18\,H_2O$ werden in 250 ml Wasser gelöst und 2 g Tartrazin-Farbstoff zugegeben. Unter Rühren werden 65 ml NaOH (50%ig) zugegeben. Der voluminöse Niederschlag wird bei 110°C getrocknet. Nach Feinmahlung des Trockenkuchens wird bei 300°C 5 min geglüht. Das Produkt wird mit 0,1 l n $H_3PO_4$ gewaschen. Nach Trocknung verbleibt ein in Wasser oder künstlichem Magensaft weitgehend unlösliches gelbes Farbmittel.

Beispiel 3

100 g $Al_2(SO_4)_3 \cdot 18\,H_2O$ werden in 120 ml Wasser gelöst und 1 g Eriochromschwarz T zugegeben. Unter Rühren werden 50 ml NaOH (50%ig) zugegeben. Der Niederschlag wird bei 110°C getrocknet und bei 390°C 5 min geglüht. Nach Waschen mit 1 n HCL verbleibt ein in Wasser bzw. Magensaft unlösliches schwarz-blaues

Farbmittel.

**Patentanspruch**

Verfahren zur Herstellung von wasserunlöslichen organischen Farbstoffen, insbesondere zur Verwendung als Kosmetik-, Lebensmittel- und Arzneimittel-Farbstoffe, mit in kristallines Aluminiumoxid eingelagerten wasserlöslichen organischen Farbstoffen, dadurch gekennzeichnet, daß aus einer Lösung eines Aluminiumsalzes mit dem Farbstoff Aluminiumhydroxid ausgefällt und der abgetrennte Niederschlag bei Temperaturen zwischen 250 und 500° C geglüht wird.

**Claim**

A process for the production of water-insoluble organic dyes, in particular for use as cosmetic, food and drug dyes, using watersoluble organic dyes which are embedded in crystalline aluminium oxide, characterized in that aluminium hydroxide is precipitated from a solution of an aluminium salt with the dye and the precipitate which is separated is annealed at a temperature of from 250 to 500° C.

**Revendication**

Procédé pour la préparation de colorants organiques insolubles dans l'eau, notamment pour des applications comme colorants pour cosmétiques, denrées alimentaires et produits pharmaceutiques, avec des colorants organiques solubles dans l'eau incorporés dans de l'oxyde d'aluminium cristallisé, procédé caractérisé en ce que, à partir d'une solution d'un sel d'aluminium avec le colorant, on fait précipiter de l'hydroxyde d'aluminium et l'on calcine le précipité séparé à une température comprise entre 250 et 500° C.